(19) Europäisches Patentamt
European Patent Office
Office européen des brevets

(11) Publication number: 0 085 234 B1

(12) **EUROPEAN PATENT SPECIFICATION**

(45) Date of publication of patent specification: 26.02.86

(21) Application number: 82306427.4

(22) Date of filing: 03.12.82

(51) Int. Cl.[4]: C 07 C 1/20, C 07 C 1/24, C 07 C 11/04, C 07 C 11/06, B 01 J 29/04, B 01 J 29/06, B 01 J 29/38

(54) **Catalytic process for light olefin production and process for catalyst regeneration.**

(30) Priority: 30.12.81 US 335796
30.12.81 US 335797

(43) Date of publication of application: 10.08.83 Bulletin 83/32

(45) Publication of the grant of the patent: 26.02.86 Bulletin 86/09

(84) Designated Contracting States: BE DE FR GB IT NL

(56) References cited:
EP-A-0 009 894
US-A-4 079 095
US-A-4 079 096

(73) Proprietor: MOBIL OIL CORPORATION
150 East 42nd Street
New York New York 10017 (US)

(72) Inventor: Forbus, Nancy Page
80 Colonial Drive
Newtown Pennsylvania 18947 (US)
Inventor: May-Som Wu, Margaret
7 Warrenton Way
Belle Mead New Jersey 08802 (US)

(74) Representative: West, Alan Harry et al
Mobil Court 3 Clements Inn
London WC2A 2EB (GB)

EP 0 085 234 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for converting methanol and/or dimethyl ether into light olefins over crystalline aluminosilicate zeolite catalysts, and to a process for regenerating such catalysts.

A remarkable growth in the production of synthetic fibers, plastics and rubber has taken place in recent decades. Such growth has been supported and encouraged to a large extent by an expanding supply of inexpensive petroleum raw materials such as ethylene and propylene. However, increasing demand for these light olefins has led to periodic shortages, due either to a diminished supply of suitable feedstocks or to limited processing capacity. In any event, it is now considered highly desirable to provide efficient means for converting raw materials other than petroleum into light olefins.

One such non-petroleum source of light olefins is coal-derived methanol and dimethyl ether. In this respect, it is known that methanol and dimethyl ether can be catalytically converted into olefin-containing hydrocarbon mixtures by contact under certain conditions with particular types of crystalline zeolite catalyst material. U.S. Patents 4,025,575, and 4,083,889 for example, both disclose processes whereby methanol and/or dimethyl ether can be converted into an olefin-containing product over a ZSM-5 type (Constraint Index 1—12) zeolite catalyst. ZSM-5, in fact, converts methanol and/or dimethyl ether into hydrocarbons containing a relatively high concentration of light ($C_2$ and $C_3$) olefins with prolonged catalyst lifetime before catalyst regeneration becomes necessary.

It is also known that other types of zeolite catalysts can be used to convert methanol and/or dimethyl ether into olefin-containing hydrocarbon products containing even higher proportions of light olefins than can be realized by methanol/dimethyl ether conversion over ZSM-5. For example, U.S. Patents 4,079,095 and 4,079,096 disclose that zeolites of the erionite-offretite type, and especially ZSM-34, can usefully be employed to promote conversion of methanol and/or dimethyl ether into products comprising a major amount of $C_2$ and $C_3$ light olefins. However, while erionite-offretite type catalysts are highly selective to light olefins production, such smaller pore zeolites tend to age rapidly in comparison to ZSM-5 when used for methanol/dimethyl ether conversion. There is therefore a continuing need to develop new catalytic procedures suitable for selectively converting an organic charge comprising methanol and/or dimethyl ether into light olefin products with both high light olefin selectivity and enhanced catalyst lifetime.

Aged methanol/dimethyl ether conversion catalysts of this type can, of course, be regenerated in a conventional manner by contacting the catalyst at elevated temperature with an oxygen-containing gas such as air to effect controlled burning of coke from the deactivated catalyst. While such a conventional regeneration procedure can restore catalytic activity diminished by coke formation on the catalyst during methanol/dimethyl ether conversion, regeneration in this manner must be conducted in the absence of organic reactants and preferably in a separate regeneration zone remote from the methanol/dimethyl ether conversion zone. Furthermore, catalyst regeneration by controlled burning of coke produces water and carbon dioxide, and water at high temperatures can permanently destroy the structure of the zeolite catalyst and can actually diminish catalytic activity in some instances. There is, therefore, a continuing need to develop additional catalyst regeneration procedures which can be employed to restore the diminished activity of the zeolite-based catalysts used to promote the conversion of methanol and/or dimethyl ether to hydrocarbon products selectively enriched in light olefins.

The present invention seeks to provide a process for the production of light olefins from methanol and/or dimethyl ether over a catalyst having a high selectivity towards light olefins and having an enhanced lifetime, and also a process for regenerating such a catalyst once its activity has been diminished by prolonged use in such a process.

According to the invention, there is provided a process for converting a feedstock comprising methanol, dimethyl ether or a mixture thereof into a hydrocarbon product rich in ethylene and propylene, which process comprises contacting the feedstock and a gaseous diluent at a temperature from 200 to 500°C, a pressure from 440 to 3550 kPa and a weight hourly space velocity for the organic reactants from 0.05 to 30, in a reaction zone with a catalyst comprising a crystalline aluminosilicate zeolite material having a crystalline structure having pore windows formed by 8-membered rings of oxygen atoms, characterized in that the gaseous diluent comprises a hydrogen-containing gas and is fed to the reaction zone at a weight hourly space velocity from 0.003 to 20.

The invention also provides a process for regenerating the catalytic activity of the catalyst used in such a process, which comprises contacting the catalyst with a hydrogen-containing gas at a temperature from 200 to 600°C and a pressure from 440 to 4930 kPa.

U.S. Patent 4,079,096, mentioned above, refers to the possible use of carrier gases or diluents in the reaction zone, and mentions hydrogen, carbon monoxide, carbon dioxide and nitrogen in this context, suggesting that such materials are employed in a purely passive capacity, i.e. as inert diluents or carriers. Indeed, none of the specific examples of the process of that document involves the use of such diluents, reinforcing the view that such diluents do not participate in the catalytic reaction.

That same U.S. Patent refers also to the conventional zeolite catalyst regeneration technique of burning deactivating coke from the catalyst in an oxygen-containing atmosphere. Although EP—A—0 009 894 describes the hydrogen-regeneration of a zeolite catalyst, that regeneration procedure is quite distinct from that of the present invention in that not only is it applied to an intermediate pore size zeolite but also it is designed to achieve selective and not total removal of coke from the zeolite.

Methanol and/or dimethyl ether can be converted into hydrocarbons in accordance with the invention by contacting such reactants with a particular type of crystalline aluminosilicate zeolite catalyst material. Such zeolites have a crystal structure that provides constrained access to, and egress from, the intracrystalline free space by virtue of having a pore dimension which is usually greater than about 3.6×3.7 Angstroms. Such zeolites also generally have a Constraint Index substantially greater than 12. Zeolitic materials of this type have pore windows of about the size provided by 8-membered rings of oxygen atoms. It is to be understood that these rings are those formed by the regular disposition of the $AlO_4$ and $SiO_4$ tetrahedra making up the anionic framework of the crystalline aluminosilicate zeolite, the oxygen atoms themselves being bonded to silicon or aluminum atoms at the centers of the tetrahedra.

These zeolites useful herein include zeolite types which may contain some crystalline zeolitic material having pore windows of a size formed by oxygen atom rings containing more than 8 members. For example, a number of natural and synthetic zeolites are known to comprise intergrowths or more than one type of crystalline material. Thus, a given zeolite may contain some crystalline material which has pore windows formed by 8-membered rings of oxygen atoms and some material having pore windows formed by 10- or 12-membered rings. The zeolites employed in the process of the invention are those which have at least a portion of their total crystalline zeolitic material composed of zeolite material having pore windows formed by 8-membered rings of oxygen atoms.

Zeolites which comprise at least some of the 8-membered ring crystalline zeolite material include those of the erionite-offretite family such as synthetic and natural erionite, synthetic and natural offretite, Zeolite T, Zeolite W, natural and synthetic chabazite and ZSM-34. Chabazite, erionite and offretite are all more particularly described in Meier and Olson, *Atlas of Zeolite Structure Types*, published in 1978 by the International Zeolite Association and elsewhere. Zeolite T is described in U.S. Patent 2,950,952 and Zeolite W is described in U.S. Patent 3,012,853.

A particularly preferred zeolite material for use in the catalyst compositions of the invention is ZSM-34. ZSM-34 and its synthesis are more fully described in U.S. Patents 4,116,813 and 4,086,186.

ZSM-34 is a unique crystalline aluminosilicate zeolite, belonging to the erionite-offretite family, having the composition (expressed in terms of molar proportions of constituent oxides) as synthesized, and after drying of:

$$(0.5\text{—}1.3)R_2O:(0\text{—}0.15)Na_2O:(0.10\text{—}0.50)K_2O:Al_2O_3:\times SiO_2$$

where R is the organic nitrogen-containing cation derived from choline $[(CH_3)_3NCH_2CH_2OH]$ and X is 8 to 50, preferably 8 to 30 and more preferably 8 to 20. This zeolite, unlike other members of the erionite-offretite family, appears to have a tabular morphology and the capability, after calcination at 540°C for at least a period of time sufficient to remove the organic cation, of sorbing at least 9.5 weight percent of n-hexane at ambient temperature and a n-hexane pressure of 2.6 kPa which is higher than that for any other known offretite or erionite. ZSM-34 is characterized by the X-ray powder diffraction pattern set forth in U.S. Patents 4,116,813 and 4,086,186.

The above zeolites, as synthesized, may be calcined to remove the organic constituent ($R_2O$) and/or ion exchanged to replace the alkali metal ions with hydrogen ion precursor, for example ammonium, and/or other metal ions, particularly metals from Groups IV, IIA, IIV, IIIB, VIIA, VIII and the rare earth metals with only minor changes in the X-ray characterization and sorption properties. The ion exchanged products are catalytically active zeolites useful in the process of this invention.

For the purposes of the process of the invention, it may be desirable to incorporate the crystalline aluminosilicate zeolites in another material resistant to the temperature and other conditions employed in the process. Such matrix materials include synthetic and naturally-occurring substances as well as inorganic materials such as clay, silica and/or metal oxides. The latter may be either naturally occurring or in the form of gelatinous precipitates or gels including mixtures of silica and metal oxides. Naturally occurring clays which can be composited with the zeolite include those of the montmorillonite and kaolin families, including the sub-bentonites and the kaolins commonly known as Dixie, McNamee-Georgia and Florida clays or others in which the main mineral constituent is halloysite, kaolinite, dickite, nacrite or anauxite. Such clays can be used in the raw state as originally mined or initially subjected to calcination, acid treatment or chemical modification.

In addition to the above materials, the small pore zeolites may be compounded with a porous matrix material, such as alumina, silica-alumina, silica-magnesia, silica-zirconia, silica-thoria, silica-beryllia, silica-titania, silica-alumina-thoria, silica-alumina-zirconia, silica-alumina-magnesia or silica-magnesia-zirconia. The matrix may be in the form of a cogel. The relative proportions of finely divided zeolite and inorganic oxide gel matrix may vary widely with the zeolite content ranging from 1 to 99 percent by weight and more usually from 5 to 80 percent by weight of the composite.

The process of the invention involves utilization of these catalyst compositions to promote the selective conversion of methanol and/or dimethyl ether into hydrocarbons, particularly light ($C_2$—$C_3$) olefins. Processes of this general type are described more fully in U.S. Patents 4,079,095 and 4,079,096.

In accordance with the invention, a chargestock comprising methanol, dimethyl ether, methanol/dimethyl ether mixtures or mixtures of such organic reactants with water is contacted in the vapor phase with the particular catalyst materials described above under reaction conditions suitable for effecting

conversion of methanol and/or dimethyl ether into olefins. When water is employed along with the organic feed, the amount of water fed with the organic charge of methanol and/or dimethyl ether is generally at least 0.25 moles of water per mole of the organic reactants. Preferably, the amount of water is greater than 0.5 moles of water per mole of organic reactants. The amount of water initially added to the organic charge usually will not exceed 40 moles per mole of said charge.

In accordance with the invention, the conversion is carried out in the presence of a gaseous diluent which provides a reducing atmosphere in the conversion reaction zone. The gaseous diluent co-fed to the reaction zone along with the organic reactant(s) to provide such a reducing atmosphere comprises a hydrogen-containing gas which can be selected from hydrogen and mixtures of hydrogen and carbon monoxide, for example those found in synthesis gas. The gaseous diluent can be co-fed at a weight hourly space velocity (WHSV) of from 0.003 to 20, preferably from 0.01 to 10. Generally the molar ratio of gaseous diluent to the organic reactants is from 0.5:1 to 40:1, preferably from 1:1 to 20:1.

When mixtures of hydrogen and carbon monoxide are used as the gaseous diluent, the molar ratio of hydrogen to carbon monoxide can vary from 0.2:1 to 10:1, preferably from 0.5:1 to 3:1. One source of such hydrogen/carbon monoxide mixtures is synthesis gas from petroleum or coal processing. Synthesis gas for use as the gaseous diluent in the process of this invention may therefore consist of a mixture of various gases such as hydrogen, carbon monoxide, carbon dioxide, methane, nitrogen, carbonyl sulfide, carbon disulfide, ammonia and hydrogen sulfide. Such synthesis gas may be derived from fossil fuel conversion by any of the known conversion and gasification methods. The term "fossil fuels" is intended to include anthracite and bituminous coal, lignite, crude petroleum, shale oil, oil from tar sands, natural gas, and fuels derived from simple physical separations or more profound transformations of these materials, including coked coal, petroleum coke, gas oil, residua from petroleum distillation, and any two or more such materials in combination. Other carbonaceous fuels such as peat, wood and cellulosic waste materials also may be used.

Processes for the conversion of coal and other hydrocarbons such as natural gas to a gaseous mixture consisting essentially of hydrogen and carbon monoxide, or of hydrogen, carbon monoxide and carbon dioxide, are well known. Although various processes may be employed for gasification, those of major importance depend either on the partial combustion of the fuel with an oxygen-containing gas or on the high temperature reaction of the fuel with steam, or on a combination of these two reactions. Such gasification processes include *in situ* gasification schemes, such as the underground partial combustion of coal and petroleum deposits. An excellent summary of the art of gas manufacture, including synthesis gas, from solid and liquid fuels, is given in Encyclopedia of Chemical Technology, Edited by Kirk-Othmer, Second Edition, Volume 10, pages 353—433 (1966), Interscience Publishers, New York, N.Y.

The raw synthesis gas produced from fossil fuels will contain various impurities such as particulates, sulfur compounds, and metal carbonyl compounds, and will be characterized by a hydrogen-to-carbon oxides ratio that will depend on the fossil fuel and the particular gasification technology utilized. Such raw synthesis gas is generally purified before being used as the gaseous diluent in the process of the invention.

Whatever the nature of particular hydrogen-containing gaseous diluent utilized, it has been found that gaseous diluents of the type described above can usefully be employed to prolong the lifetime of zeolite-based catalysts when such catalysts and diluents are employed under particular conditions for the selective conversion of methanol/dimethyl ether into light olefins. Such conditions include an operating temperature of from 200 to 500°C, preferably 300 to 450°C, a pressure of from 440 kPa to 3550 kPa, preferably 680 kPa to 1830 kPa; and a weight hourly space velocity (WHSV) of the organic reactants of from 0.05 to 30, preferably 0.1 to 10.

The methanol and/or dimethyl ether conversion described above may be carried out as a batch-type, semi-continuous or continuous operation utilizing a fixed, fluidized or moving bed catalyst system. A preferred arrangement entails use of a catalyst zone in which the alcohol or ether charge together with gaseous diluent and optionally added water is passed cocurrently or countercurrently through a fluidized or moving bed of particulate catalyst.

The product stream from the process of the invention contains steam and a hydrocarbon mixture of paraffins and olefins, substantially devoid of aromatics. This mixture is particularly rich in light olefins (ethylene and propylene). Generally, a major fraction of the total olefins is ethylene plus propylene with the ethylene content of the product exceeding the propylene content. Thus, the predominant hydrocarbon product constitutes valuable petrochemicals. The steam and hydrocarbon products may be separated from one another by methods well known in the art. Preferably, the unconverted methanol and/or dimethyl ether, as well as at least part of the water in the product, can be recycled to the reaction zone.

The catalyst regeneration conditions employed according to the invention include a regeneration temperature of from 200 to 600°C, preferably from 300 to 500°C and a regeneration pressure of from 440 kPa to 5000 kPa, preferably from 780 kPa to 3550 kPa. Regeneration may be carried out by conducting the aged catalyst to a separate regeneration zone for contact with hydrogen-containing gas, after which the regenerated catalyst is recycled to the conversion zone for further contact with the methanol- and/or dimethyl ether-containing feed. Alternatively, the aged catalyst can be regenerated in the reaction zone itself by controlled contact with hydrogen-containing gas under the above regeneration conditions.

Preferably, however, the catalyst is regenerated by contacting the catalyst with hydrogen-containing gas under the above regeneration conditions in the substantial absence of the organic reactants. Since a

hydrogen-containing diluent is used in the conversion, regeneration is conveniently accomplished by simply discontinuing the flow of organic reactants to the reaction zone while allowing flow of hydrogen-containing diluent to continue into the reaction zone under conditions effective to bring about the desired catalyst regeneration.

Regeneration of the methanol/dimethyl ether conversion catalyst can also be carried out in the presence of the organic reactants used in the conversion reaction. Regeneration of this type can be effected by adding hydrogen-containing gas to the reactant-containing feedstream introduced into the reaction zone or by increasing pressure in the reaction zone which already contains the organic reactants and hydrogen-containing gas being used as a diluent. To bring about restoration of diminished catalytic activity in such instances where hydrogen is already present during the methanol/dimethyl ether conversion reaction, it is generally necessary to contact the catalyst with hydrogen-containing gas at a regeneration pressure which exceeds the elevated pressure of the methanol/dimethyl ether conversion conditions existing in the reaction zone immediately prior to catalyst regeneration.

Catalyst regeneration using hydrogen-containing gas in the manner described above produces no water or carbon dioxide as by-products of the regeneration procedure. Regeneration can thus be effected without the possibility of forming potentially catalyst-damaging amounts of water.

The following Examples illustrate the invention.

## Example I

ZSM-34 was prepared by interacting the following:

A. Caustic aluminate
68.89 grams sodium aluminate (20 wt.% Na, 43.1 wt.% $Al_2O_3$, balance $H_2O$)
29.28 grams NaOH (77.5 wt.% $Na_2O$)
26.4 grams KOH (86.4% $K_2O$)
540 grams $H_2O$

B. Silica solution
780 grams colloidal silica sol (30 wt.% $SiO_2$)

C. Choline chloride
228 grams

These were mixed together in a 2 liter autoclave, adding C to A and then adding B followed by 15 minutes continuous mixing. The autoclave was sealed, pressure-tested and then heated to and held at 150°C for 8 days. The contents were stirred continuously during the 8 day crystallization period.

The autoclave and its contents were then cooled to room temperature, and the crystalline product was filtered off and washed. On analysis the product was found to contain:

Na, wt.%: 0.68
K, wt.%: 3.59
$Al_2O_3$ wt.%: 13.5
$SiO_2$, wt.%: 78.5
N, wt.%: 2.5

The resulting ZSM-34 product had the following molar composition:

$$0.54\ R_2O:0.11\ Na_2O:0.35\ K_2O:Al_2O_3:9.87\ SiO_2$$

A sample of the calcined alkali ZSM-34 was further processed by contact with a 10 wt.% $NH_4Cl$ solution for 1 hour at about 85°C using 10 ml of solution for each gram of ZSM-34. A total of four contacts were made at these conditions followed by final filtration and water washing essentially free of chloride ion.

The product was dried at 110°C and calcined for 10 hours at 540°C. The residual alkali content as Na was 0.035 wt.% while the residual K content was 1.47 wt.%. This product had a surface area of 517 $m^2/g$ and the following sorption capacities:

Cyclohexane, wt.%: 2.6
n-Hexane, wt.%: 10.0
$H_2O$, wt.%: 18.7

## Example II

ZSM-34 prepared in a manner similar to that of Example I and back-exchanged with ammonium chloride to convert it into the ammonia form was used to convert methanol into hydrocarbons in known manner. The ZSM-34 used in such conversion had a surface area of 475 $m^2/g$ and the following sorption capacities:

Cyclohexane, wt.%: 4.5
n-Hexane, wt.%: 9.9
$H_2O$, wt.%: 16.5

In such a procedure, two grams of zeolite (no binder) and four grams of quartz chips, both of 14/20 mesh size, were mixed and packed into a quartz microreactor equipped with a thermocouple. Several cycles were run, and the catalyst was always calcined at 500°C with air for at least 16 hours before each new cycle. The standard feed contained 37.2% MeOH and 62.8% $H_2O$ (by weight). The methanol/water mixture was fed to the reactor maintained at 370°C/100 kPa using a weight hourly space velocity (WHSV) of 4.1. The total reactor effluent was analyzed on line by a "n-octane on Poracil" column. Methanol conversion was calculated based on hydrocarbon formation only. Selectivities (wt.%) to hydrocarbon product were calculated on "coke free" basis.

The lifetimes for converting 50% of the methanol for each cycle and the corresponding selectivities to $C_2H_4$, $C_3H_6$ and $C_4H_8$ are summarized in Table I.

TABLE I

Catalyst lifetimes and selectivities to $C_2H_4$, $C_3H_6$ and $C_4H_8$ by $NH_4$ZSM-34 at 50% MeOH conversion

| Cycle | 1 | 2 | 3 | 4 |
|---|---|---|---|---|
| Lifetime for 50% MeOH conversion, (hours) | 2.6 | 2.0 | 1.7 | 1.4 |
| Selectivities (wt.%) at 50% MeOH conversion | | | | |
| $C_2H_4$ | 61 | 56 | 58 | 55 |
| $C_3H_6$ | 25 | 26 | 29 | 28 |
| $C_4H_8$ | 4 | 7 | 7 | 7 |
| Total $C_2$=—$C_4$= | 90 | 89 | 94 | 90 |

The Table I data demonstrate that $NH_4$ZSM-34 provides relatively high selectivity to light olefins for conversion of a methanol/water feed into hydrocarbons. Such data also indicate that catalyst lifetime for methanol conversion over $NH_4$ZSM-34 is relatively short.

Example III

Another sample of a ZSM-34 zeolite was used to promote conversion of an anydrous methanol feed into hydrocarbons using the hydrogen co-feed and elevated temperature and pressure conditions of the invention. In such a procedure, a fresh two gram sample of $NH_4$ZSM-34 in the same reactor used in Example II was purged with $H_2$ at 100 cc/min, 350°C and 960 kPa for 6 hours. Methanol was then introduced at 1 cc/hr. The first sample was taken after 24 hours on stream at 350°C and 960 kPa with methanol and hydrogen feeds at WHSV values of 0.4 and 0.2, respectively. The reactor pressure and methanol and hydrogen feed rates were maintained constant throughout the run. For the first 29 hours, when the reactor temperature was 353°C to 365°C, methanol conversion was below 50%. When the reactor temperature was raised to 375°C, methanol conversion increased to above 65%. The reaction conditions were then maintained constant for 120 hours. The major by-products were $CH_4$, $C_2H_6$ and $C_3H_8$. The run was terminated arbitrarily after a total of 148 hours. The reaction conditions and average results for this run are summarized below:

| | |
|---|---|
| Temperature | 375°C |
| Pressure | 960 kPa |
| WHSV of MeOH | 0.4 |
| WHSV of $H_2$ | 0.2 |
| Methanol conversion | 75% |
| Selectivity (wt.%) | |
| $C_2H_4$ | 55 |
| $C_3H_6$ | 24 |
| $CH_4$ | 7 |
| $C_2H_6$ | 7 |
| Others | 7 |
| Total $C_2^=$—$C_4^=$ | 81 |

Example IV

Another continuous series of runs employing $NH_4$ZSM-34 zeolite catalyst were carried out in order to convert an anhydrous methanol feed into hydrocarbon product in the same reactor used in Examples II and III. Reaction conditions were varied for each run. Except as noted, no regeneration procedure was employed between runs. Reaction conditions, methanol conversion and light olefins selectivity for each run are set forth in Table II.

TABLE II

Effect of reaction variables on methanol conversion over ZSM-34

| Run No. | 1 | 2 | 3 | 4 | 5 | 6 | 7 | 8 | Catalyst is regenerated by $H_2$ overnight at 350°C, 790 kPa, 100 cc/min. | 9 | 10 | 11 | 12 |
|---|---|---|---|---|---|---|---|---|---|---|---|---|---|
| Total time on stream (hrs) | 2 | 24 | 44 | 71 | 91 | 94 | 120 | 184 | | 25 | 80 | 125 | 167 |
| Temperature, °C | 394 | 394 | 380 | 370 | 370 | 365 | 365 | 365 | | 370 | 370 | 370 | 370 |
| Pressure, kPa | 3550 | 1825 | 1825 | 1825 | 1825 | 790 | 790 | 790 | | 790 | 1480 | 1135 | 960 |
| WHSV: $H_2$ | 0.2 | 0.2 | 0.2 | 0.2 | 0.1 | 0.1 | 0.2 | 0.2 | | 0.2 | 0.2 | 0.2 | 0.2 |
| MeOH | 0.4 | 0.4 | 0.4 | 0.4 | 0.4 | 0.4 | 0.4 | 0.8 | | 0.4 | 0.4 | 0.4 | 0.4 |
| MeOH conversion, %*** | 100 | 100 | 98 | 66 | 54 | 38 | 41 | 21 | | 50** | 70 | 61 | 54 |
| Wt.% selectivity $C_2H_4$ | 10 | 33** | 28 | 41 | 43 | 57 | 57 | 49 | | 58 | 40 | 48 | 50 |
| $C_3H_6$ | 13 | 21 | 27 | 24 | 24 | 24 | 24 | 26 | | 25 | 25 | 25 | 25 |
| $C_4H_8$ | 12.0 | 10 | 0 | 1 | 0 | 0 | 0 | 0 | | 0 | 0 | 2 | 4 |
| $CH_4$ | 10.0 | 11 | 7 | 10 | 12 | 7 | 7 | 9 | | 7 | 14 | 11 | 10 |
| $C_2H_6$ | 18 | —* | 11 | 10 | 10 | 5 | 4 | 4 | | 7 | 13 | 10 | 10 |
| $C_3H_8$ | 34.0 | 19 | 15 | 10 | 5 | 3 | 1 | 1 | | 2 | 8 | 5 | 2 |
| Others | 3 | 6 | 12 | 4 | 6 | 4 | 7 | 11 | | 1 | 0 | 0 | 0 |
| Total $C_2$—$C_4$= | 35.0 | — | 55 | 66 | 67 | 81 | 81 | 75 | | 83 | 65 | 75 | 80 |

*$C_2$, $C_2$=were not separated.
**Methanol conversion never reached a steady state under this set of conditions.
***To hydrocarbons.

The Table II data demonstrate the effect of temperature, hydrogen and methanol flow rates and reactor pressure on methanol conversion and light olefin selectivity. In Runs 2, 3 and 4 of Table II, the reactor temperature was varied from 394 to 370°C at a constant pressure of 1825 kPa. The methanol conversion decreased from 100% to 66% and selectivities to ethylene and total light olefins were significantly improved at lower temperature.

Comparing Runs 4 and 5, when the hydrogen feed rate was halved, the steady state methanol conversion decreased from 66% to 54% and selectivities to light olefins remained similar. In Run 8, when the methanol feed rate was doubled, compared to Run 7, the methanol conversion dropped from 41% to 21%.

In Run 9 to 12, the effect of reactor pressure was demonstrated by maintaining reactor temperature at 370°C and WHSV of $H_2$ and MeOH at 0.2 and 0.4, respectively. The reactor pressure was varied from 790 to 1480 kPa. It is important to note that the catalyst maintained good activity and selectivities to $C_2$ and $C_3$ olefins over a wide range of reactor pressures.

Example V

Another run employing somewhat different reaction conditions from those of Examples III and IV was carried out to convert methanol into hydrocarbons over $NH_4$ZSM-34 zeolite catalyst. In such testing, 10 grams of undiluted zeolite (14/20 mesh) were used in a stainless steel vapor phase reactor fitted with a furnace and both water and dry ice condensers. Both a liquid water phase from the condensers and uncondensed gases were collected and analyzed. The volume of hydrocarbons was determined by subtracting the volume of hydrogen feed from the total volume of non-condensed gas obtained. The liquid water phase was analyzed for water, methanol and dimethyl ether on a 3.2 mm×915 mm Poropak T column. The gases were analyzed on an 3.2 mm×2440 mm Silica Gel column (Analabs) 100/120 mesh. Material balances were calculated from the liquid and two gaseous samples analyzed.

The catalyst in the reactor was heated in a flowing hydrogen steam for 1.5 hours at 500°C before introduction of the methanol reactant. Methanol was then introduced with the hydrogen with the temperature lowered to 300°C. Conversion of methanol then continued for 205 hours in a series of runs with varying temperature, pressure, space velocity and molar $H_2$/methanol feed ratios. Reaction conditions, methanol conversion and product selectivities for the series of runs are set forth in Tables IIIA and IIIB.

TABLE IIIA
Methanol to olefins—ZSM-34
runs 1—7

| Conditions | Run No. 1 | 2 | 3 | 4 | 5 | 6 | 7 |
|---|---|---|---|---|---|---|---|
| Temp., °C | 300 | 350 | 360 | 370 | 370 | 370 | 370 |
| Pressure, kPa | 960 | 960 | 960 | 960 | 960 | 960 | 960 |
| WHSV MeOH | .43 | .43 | .43 | .43 | .72 | .46 | .46 |
| $H_2$ | .26 | .26 | .26 | .26 | .27 | .21 | .12 |
| Mole ratio | 1/10 | 1/10 | 1/10 | 1/10 | 1/6 | 1/7.5 | 1/4 |
| Time on stream (hrs) | 1.5 | 11 | 21 | 32 | 44 | 67 | 80 |
| Conversion (%) | 99 | 91 | 89 | 100 | 86 | 90 | 71 |
| Select. to prods. (%) $C_2H_4$ | 14.8 | 33.5 | 41.7 | 36.6 | 42.1 | 40.2 | 41.9 |
| $C_3H_6$ | 15.9 | 25.2 | 28.1 | 31.3 | 29.1 | 29.1 | 29.9 |
| $C_4H_8$ | 12.0 | 11.0 | 5.5 | 4.7 | 4.4 | 5.7 | 6.1 |
| Total | 42.7 | 69.7 | 75.3 | 72.6 | 75.6 | 75.0 | 77.9 |
| $CH_4$ | 28.1 | 8.9 | 10.8 | 9.6 | 14.5 | 10.8 | 8.3 |
| $C_2H_6$ | 2.4 | 7.4 | 8.3 | 8.9 | 7.1 | 8.1 | 8.1 |
| $C_3H_8$ | 17.9 | 8.0 | 4.2 | 5.6 | 3.7 | 4.0 | 2.7 |
| $C_4H_{10}$ | 6.4 | 1.9 | .3 | .3 | .2 | .2 | .3 |
| Total | 54.8 | 26.2 | 23.6 | 24.4 | 22.5 | 23.1 | 19.4 |
| CO, $CO_2$, $C_5$+ | 2.5 | 4.1 | 1.1 | 2.7 | 1.9 | 1.9 | 2.7 |
| TOTAL | 100.0 | 100.0 | 100.0 | 100.0 | 100.0 | 100.0 | 100.0 |

TABLE IIIB

Methanol to olefins ZSM-34 runs 8—14

| Conditions | Run No. 8 | 9 | 10 | 11 | 12 | 13 | 14 |
|---|---|---|---|---|---|---|---|
| Temp., °C | 390 | 400 | 400 | 390 | 390 | 380 | 380 |
| Pressure, kPa | 960 | 960 | 1135 | 1135 | 1310 | 1310 | 1480 |
| WHSV MeOH | .43 | .46 | .44 | .44 | .44 | .44 | .44 |
| $H_2$ | .12 | .13 | .12 | .12 | .12 | .12 | .12 |
| Mole ratio | 1/4.5 | | 1/4.3 | 1/4.3 | 1/4.3 | 1/4.3 | 1/4.3 |
| Time on stream (hrs) | 110 | 134 | 149 | 158 | 168 | 180 | 205 |
| Conversion (%) | 94 | 96 | 100 | 91 | 96 | 83 | 94 |
| Select. to prods. (%) $C_2H_4$ | 40.7 | 42.9 | 36.2 | 41.5 | 35.2 | 39.0 | 33.9 |
| $C_3H_6$ | 30.2 | 29.6 | 26.5 | 28.7 | 28.2 | 26.5 | 26.9 |
| $C_4H_8$ | 6.7 | 4.7 | 5.3 | 4.3 | 5.7 | 5.0 | 4.1 |
| Total | 77.6 | 77.2 | 68.0 | 74.5 | 69.1 | 70.5 | 64.9 |
| $CH_4$ | 8.5 | 9.1 | 9.5 | 9.9 | 11.2 | 11.6 | 11.5 |
| $C_2H_6$ | 7.3 | 7.9 | 11.4 | 9.7 | 12.6 | 11.9 | 15.5 |
| $C_3H_8$ | 2.4 | 2.1 | 4.1 | 2.6 | 3.5 | 3.2 | 5.5 |
| $C_4H_{10}$ | .4 | .3 | .7 | .2 | .3 | .3 | .4 |
| Total | 18.6 | 19.4 | 25.7 | 22.4 | 27.6 | 27.0 | 32.9 |
| CO, $CO_2$, $C_5$+ | 3.8 | 3.4 | 6.3 | 3.1 | 3.3 | 2.5 | 2.2 |
| TOTAL | 100.0 | 100.0 | 100.0 | 100.0 | 100.0 | 100.0 | 100.0 |

The Table IIIA and IIIB data demonstrate the beneficial effect on conversion and light olefin selectivity of co-feeding hydrogen with methanol when a ZSM-34 zeolite is used to convert methanol into light olefins at temperature of from 300 to 400°C and pressure of from 960 kPa to 1480 kPa.

Example VI

A two gram sample of $NH_4$ZSM-34, diluted with four grams of quartz chips (both of 14/20 mesh), was used to convert methanol into hydrocarbons in the reactor of Example II. The reactor was pressurized to 3550 kPa with 1/1 (v/v) $CO/H_2$. The reactor temperature was raised to 395°C and methanol was fed at 1.0 ml/hr. The $CO/H_2$ feed rate was maintained at 100 cc/min. Results are given in Table IV.

TABLE IV

Methanol conversion over $NH_4ZSM$-34 under $CO/H_2$ pressure

| Temp. (°C) | Time on stream (hr) | % conv. | Hydrocarbon product selectivity | | | | | | |
|---|---|---|---|---|---|---|---|---|---|
| | | | $C_1°$ | $C_2°$ | $C_2=$ | $C_3°$ | $C_3=$ | $C_4$ | Others |
| 395 | 1.0 | 90 | 5.1 | 6.8 | 34.3 | 27.2 | 26.7 | — | — |
| 395 | 2.0 | 90 | 6.5 | 9.5 | 41.6 | 8.9 | 33.8 | — | — |
| 395 | 3.5 | 92.1 | 6.2 | 10.5 | 41.5 | 10.2 | 24.5 | 3.9 | 3.2 |
| 395 | 19.5 | 98.9 | 7.3 | 11.3 | 35.9 | 8.2 | 24.7 | 2.9 | 9.7 |

The Table IV data demonstrate that, by using an $NH_4ZSM$-34 catalyst and a $CO/H_2$ co-feed, methanol can be effectively converted into hydrocarbons with high selectivity to light olefins even after 19 hours on stream.

Example VII

Without any regeneration procedure, the gas feed in the Example IV reaction was changed to pure $H_2$ and the methanol reaction continued. Methanol conversion and product selectivities at 370°C, hydrogen flow of 100 cc/min, $CH_3OH$ feed of 1 ml/hr and various pressures are summarized in Table V.

TABLE V

Methanol conversion over $NH_4ZSM$-34 under $H_2$ pressure

| Pressure (kPa) | Time on stream (hr) | % conv. | Hydrocarbon product selectivity* | | |
|---|---|---|---|---|---|
| | | | $C_2=$ | $C_3=$ | $C_2=$—$C_4=$ |
| 790 | 4.5 | 77.1 | 52.7 | 26.5 | 83.0 |
| 790 | 21.9 | 37.3 | 52.3 | 25.5 | 80.4 |
| 1480 | 23.5 | 51.4 | 39.8 | 23.1 | 65.6 |
| 1135 | 76.4 | 72.5 | 45.7 | 24.3 | 72.5 |
| 960 | 125.4 | 54.2 | 52.8 | 23.4 | 78.4 |
| 960 | 165.2 | 68.4 | 49.8 | 26.0 | 78.3 |

*Balance contained mostly $CH_4$, $C_2H_6$, $C_3H_8$ and $C_4H_{10}$.

The Table V data again demonstrate the beneficial effect of an $H_2$ co-feed at elevated pressure on catalyst lifetime when $NH_4ZSM$-34 zeolite is used to promote conversion of methanol into light olefins.

Example VIII

In a conversion and regeneration procedure, two grams of $NH_4ZSM$-34 as described in Example II of 14/20 mesh size and diluted with 4 g quartz chips were packed into a reactor for methanol into ethylene conversion under hydrogen pressure. After 72 hours on stream for methanol reaction under various temperatures and pressures, the reaction conditions were set at 790 kPa, 365°C with hydrogen feed rate at 100 cc/min and methanol at 1 cc/hr or 2 cc/hr. These results are summarized in Table VIA. In these runs, the selectivities to ethylene and total light olefins were high, but methanol conversion into hydrocarbons was low.

The methanol feed was then discontinued. Only hydrogen gas was fed through the catalyst for regeneration. Hydrogen feed rate was 100 cc/min. Reactor temperature was maintained at 400°C and reactor pressure at 790 kPa.

After the regeneration, reactor conditions were returned to 790 kPa, 370°C with hydrogen feed rate at 100 cc/min and methanol at 1 cc/hr. The results of such methanol conversion into hydrocarbons subsequent to regeneration are summarized in Table VIB.

TABLE VIA

Methanol conversion to light olefins by $NH_4$ZSM-34 followed by hydrogen regeneration of catalyst

| Reaction conditions before $H_2$ regeneration | Time on stream (hrs) | % $CH_3OH$ conv. | Wt.% selectivity | | | | | |
|---|---|---|---|---|---|---|---|---|
| | | | $CH_4$ | $C_2H_6$ | $C_2H_4$ | $C_3H_8$ | $C_3H_6$ | $C_2$=—$C_4$= |
| 790 kPa, 365°C | 73.50 | 31.70 | 7.10 | 4.40 | 57.20 | 1.50 | 23.50 | 80.70 |
| $H_2$, 100 cc/min | 74.50 | 30.60 | 6.90 | 4.40 | 56.80 | 1.40 | 23.70 | 80.50 |
| MeOH, 1 cc/hr | 75.50 | 34.30 | 6.70 | 4.40 | 58.20 | 1.50 | 23.00 | 81.20 |
| | 92.50 | 53.80 | 6.10 | 4.10 | 57.90 | 1.90 | 24.40 | 82.30 |
| | 95.50 | 47.00 | 6.40 | 4.00 | 58.10 | 1.50 | 24.00 | 82.10 |
| | 97.50 | 43.60 | 6.90 | 4.20 | 56.90 | 1.40 | 24.00 | 80.90 |
| 790 kPa, 365°C | 117.50 | 21.00 | 8.60 | 3.90 | 48.60 | 1.00 | 26.00 | 74.60 |
| $H_2$, 100 cc/min | 142.00 | 21.10 | 8.70 | 4.40 | 47.60 | 1.00 | 26.30 | 73.90 |
| MeOH, 2 cc/hr | 162.00 | 22.00 | 8.90 | 5.20 | 46.60 | 1.10 | 26.10 | 72.70 |

After 162 hours on stream catalyst is regenerated with $H_2$ at 400°C, 790 kPa. $H_2$=100 cc/hr for 18 hours.

TABLE VIB

Methanol conversion to light olefins by $NH_4ZSM$-34 after hydrogen regeneration of catalyst

| Reaction conditions after $H_2$ regeneration | Time on stream (hrs)[1] | % $CH_3OH$ conv. | Wt.% selectivity | | | | | |
|---|---|---|---|---|---|---|---|---|
| | | | $CH_4$ | $C_2H_6$ | $C_2H_4$ | $C_3H_8$ | $C_3H_6$ | $C_2$=—$C_4$= |
| 790 kPa, 370°C | 2.7 | 98.3 | 5.8 | 7 | 35 | 8.1 | 22.4 | 68.3 |
| $H_2$, 100 cc/min | 3.3 | 97.3 | 5.5 | 6.6 | 45.9 | 4.8 | 25.2 | 77.8 |
| MeOH, 1 cc/hr | 3.9 | 87.7 | 5.4 | 6.2 | 51.2 | 3.7 | 26.4 | 82.3 |
| | 4.5 | 77.1 | 5.7 | 6.7 | 52.7 | 3.3 | 26.5 | 83 |
| | 5 | 70.3 | 5.8 | 7.1 | 53.2 | 3.2 | 26.2 | 82.7 |
| | 6 | 63.7 | 5.8 | 7 | 54.3 | 2.9 | 26 | 83.2 |
| | 6.9 | 57.9 | 6 | 7.2 | 55.3 | 2.7 | 25.4 | 83.2 |
| | 7.9 | 55 | 6 | 7.2 | 55.9 | 2.6 | 25.1 | 83.3 |
| | 9.9 | 50.9 | 6 | 7 | 57.4 | 2.3 | 24.4 | 83.9 |
| | 12 | 46.9 | 5.9 | 6.3 | 59.2 | 2 | 23.9 | 85 |
| | 13.9 | 42.1 | 6.2 | 6.1 | 60 | 1.6 | 23.4 | 85.3 |
| | 15.9 | 39.8 | 6.5 | 6.1 | 59.9 | 1.5 | 23.3 | 85.1 |
| | 17.9 | 35.4 | 7 | 6.1 | 59.3 | 1.3 | 23.5 | 84.8 |
| | 20 | 33.6 | 7.1 | 5.8 | 59.2 | 1.2 | 23.8 | 85.1 |
| | 21.9 | 37.3 | 8.2 | 8.3 | 52.3 | 1.8 | 25.5 | 80.4 |

[1]Time on stream after regeneration.

The Tables VIA and VIB data demonstrate that treatment of the aged catalyst with hydrogen promoted catalytic activity to the same levels as original fresh catalyst for conversion of methanol into a light olefin-enriched hydrocarbon product.

Example IX

Another continuous series of runs employing $NH_4ZSM$-34 zeolite catalyst was carried out in order to convert an anhydrous methanol feed into hydrocarbon product in the presence of hydrogen and with hydrogen regeneration of the catalyst. Various reaction conditions were employed for the runs up to 184 hours on stream after which time the catalyst was regenerated in the absence of organic reactants. Additional runs were then carried out with variation in hydrogen pressure. Reaction conditions, methanol conversion and light olefins selectivity for each run are set forth in Table VII.

TABLE VII

Methanol conversion over ZSM-34 with hydrogen treatment to regenerate catalyst

| Run No. | 1 | 2 | 3 | 4 | 5 | 6 | 7 | 8 | Catalyst is regenerated by $H_2$ overnight at 350°C, 790 kPa, 100 cc/min | 9 | 10 | 11 | 12 |
|---|---|---|---|---|---|---|---|---|---|---|---|---|---|
| Total time on stream (hrs) | 2 | 24 | 44 | 71 | 91 | 94 | 120 | 184 | | 25 | 80 | 125 | 167 |
| Temperature, °C | 394 | 394 | 380 | 370 | 370 | 365 | 365 | 365 | | 370 | 370 | 370 | 370 |
| Pressure, kPag | 3550 | 1825 | 1825 | 1825 | 1825 | 790 | 790 | 790 | | 790 | 1480 | 1135 | 960 |
| WHSV: $H_2$ | 0.2 | 0.2 | 0.2 | 0.2 | 0.1 | 0.1 | 0.2 | 0.2 | | 0.2 | 0.2 | 0.2 | 0.2 |
| MeOH | 0.4 | 0.4 | 0.4 | 0.4 | 0.4 | 0.4 | 0.4 | 0.8 | | 0.4 | 0.4 | 0.4 | 0.4 |
| MeOH conversion, %*** | 100 | 100 | 98 | 66 | 54 | 38 | 41 | 21 | | 50** | 70 | 61 | 54 |
| Wt.% selectivity | | | | | | | | | | | | | |
| $C_2H_4$ | 10 | 33** | 28 | 41 | 43 | 57 | 57 | 49 | | 58 | 40 | 48 | 50 |
| $C_3H_6$ | 13 | 21 | 27 | 24 | 24 | 24 | 24 | 26 | | 25 | 25 | 25 | 25 |
| $C_4H_8$ | 12.0 | 10 | 0 | 1 | 0 | 0 | 0 | 0 | | 0 | 0 | 2 | 4 |
| $CH_4$ | 10.0 | 11 | 7 | 10 | 12 | 7 | 7 | 9 | | 7 | 14 | 11 | 10 |
| $C_2H_6$ | 18 | —* | 11 | 10 | 10 | 5 | 4 | 4 | | 7 | 13 | 10 | 10 |
| $C_3H_8$ | 34.0 | 19 | 15 | 10 | 5 | 3 | 1 | 1 | | 2 | 8 | 5 | 2 |
| Others | 3 | 6 | 12 | 4 | 6 | 4 | 7 | 11 | | 1 | 0 | 0 | 0 |
| Total $C_2$—$C_4$= | 35.0 | — | 55 | 66 | 67 | 81 | 81 | 75 | | 83 | 65 | 75 | 80 |

*$C_2$, $C_2$=were not separated.
**Methanol conversion never reached a steady state under this set of conditions.
***To hydrocarbons.

The Table VII data demonstrate that aged methanol conversion catalyst can be regenerated with hydrogen under regeneration conditions by simply cutting off the methanol feed. In Table VII, between Runs 8 and 9, the catalyst was regenerated with $H_2$ for 16 hours. When the methanol feed was restarted, the catalyst was active for methanol conversion, and the original selectivities to ethylene and light olefins were restored.

The Table VII data also show that the aged catalyst can be continuously regenerated during the methanol conversion reaction by increasing hydrogen pressure. This was demonstrated in Runs 9 and 10. Under the conditions shown in Run 9, the catalyst continuously aged over a period of 20 hours. At the end of this time, methanol conversion was only 33%. The reactor pressure was then raised from 790 kPa to 1480 kPa. The methanol conversion gradually increased to 70% after five hours on stream under the new conditions.

**Claims**

1. A process for converting a feedstock comprising methanol, dimethyl ether or a mixture thereof into a hydrocarbon product rich in ethylene and propylene, which process comprises contacting the feedstock and a gaseous diluent at a temperature from 200 to 500°C, a pressure from 440 to 3550 kPa and a weight hourly space velocity for the organic reactants from 0.05 to 30 in a reaction zone with a catalyst comprising a crystalline aluminosilicate zeolite having a crystalline structure having pore windows formed by 8-membered rings of oxygen atoms, characterized in that the gaseous diluent comprises a hydrogen-containing gas and is fed to the reaction zone at a weight hourly space velocity from 0.003 to 20.

2. A process according to claim 1, wherein the zeolite is erionite, offretite, chabazite, Zeolite T, or Zeolite W.

3. A process according to claim 1, wherein the zeolite is ZSM-34.

4. A process according to any one of claims 1 to 3, carried out at a temperature from 300 to 450°C and a pressure from 790 to 1825 kPa.

5. A process according to any one of claims 1 to 4, wherein the feedstock also comprises water in an amount of at least 0.25 moles per mole of organic reactants.

6. A process according to any one of claims 1 to 5, wherein the gaseous diluent is substantially pure hydrogen and wherein the weight hourly space velocity of the hydrogen is from 0.01 to 10 and the molar ratio of hydrogen to organic reactants is from 0.5:1 to 40:1.

7. A process according to any one of claims 1 to 5, wherein the gaseous diluent comprises a mixture of hydrogen and carbon monoxide having a hydrogen to carbon monoxide molar ratio from 0.2:1 to 10:1.

8. A process for regenerating the catalytic activity of the zeolite catalyst used in the process according to any one of claims 1 to 7, which comprises contacting the catalyst with a hydrogen-containing gas at a temperature from 200 to 600°C and a pressure from 440 to 4930 kPa.

9. A process according to claim 8, wherein regeneration is carried out at from 300 to 500°C and from 790 to 3550 kPa.

10. A process according to claim 8 or claim 9, wherein the hydrogen-containing regeneration gas is substantially pure hydrogen.

11. A process according to claim 8 or claim 9, wherein the hydrogen-containing regeneration gas comprises a mixture of hydrogen and carbon monoxide having a hydrogen to carbon monoxide molar ratio from 0.2:1 to 10:1.

12. A process according to claim 8 or claim 9, wherein regeneration is carried out in the presence of methanol and/or dimethyl ether at a pressure higher than that used in the process for converting the methanol and/or dimethyl ether into hydrocarbon product.

13. A process according to any one of claims 1 to 6, wherein the catalyst is subsequently regenerated by a process according to any one of claims 8 to 10.

14. A process according to any one of claims 1 to 5 and 7, wherein the catalyst is subsequently regenerated by a process according to any one of claims 8, 9 and 11.

15. A process according to any one of claims 1 to 6, wherein the catalyst is subsequently regenerated by a process according to any one of claims 8, 9 and 12.

**Patentansprüche**

1. Verfahren zur Umwandlung eines Ausgangsstoffes, der Methanol, Dimethyläther oder eine Mischung davon umfaßt, in ein Kohlenwasserstoffprodukt, das reich an Äthylen und Propylen ist, wobei das Verfahren das Kontaktieren des Ausgangsstoffes und eines gasförmigen Verdünnungsmittels bei einer Temperatur von 200 bis 500°C, einem Druck von 440 bis 3550 KPa und einer stündlichen Gewichts-Raum-Geschwindigkeit der organischen Reaktanten von 0,05 bis 30 in einer Reaktionszone mit einem Katalysator umfaßt, wobei der Katalysator einen kristallinen Aluminosilikat-Zeolith mit einer kristallinen Struktur mit Porenfenstern umfaßt, die durch 8-gliedrige Ringe von Sauerstoffatomen gebildet werden, dadurch gekennzeichnet, daß das gasförmige Verdünnungsmittel ein wasserstoffhaltiges Gas umfaßt und zu der Reaktionszone bei einer stündlichen Gewichts-Raum-Geschwindigkeit von 0,003 bis 20 zugeführt wird.

2. Verfahren nach Anspruch 1, worin der Zeolith, Erionit, Offretit, Chabazit, Zeolith T oder Zeolith W ist.

3. Verfahren nach Anspruch 1, worin der Zeolith ZSM-34 ist.

4. Verfahren nach einem der Ansprüche 1 bis 3, ausgeführt bei einer Temperatur von 300 bis 450°C und einem Druck von 790 bis 1825 KPa.

5. Verfahren nach einem der Ansprüche 1 bis 4, worin der Ausgangsstoff ebenfalls Wasser in einer Menge von mindestens 0,25 Mol pro Mol der organischen Reaktanten umfaßt.

6. Verfahren nach einem der Ansprüche 1 bis 5, worin das gasförmige Verdünnungsmittel im wesentlichen reiner Wasserstoff ist und worin die stündliche Gewichts-Raum-Geschwindigkeit des Wasserstoffs von 0,01 bis 10 und das Molverhältnis von Wasserstoff zu den organischen Reaktanten von 0,5:1 bis 40:1 betragen.

7. Verfahren nach einem der Ansprüche 1 bis 5, worin das gasförmige Verdünnungsmittel eine Mischung von Wasserstoff und Kohlenmonoxyd mit einem Molverhältnis von Wasserstoff zu Kohlenmonoxyd von 0,2:1 bis 10:1 umfaßt.

8. Verfahren zur Regenerierung der katalytischen Aktivität des Zeolith-Katalysators, der in dem Verfahren nach einem der Ansprüche 1 bis 7 verwendet wird, welches das Kontaktieren des Katalysators mit einem wasserstoffenthaltenden Gas bei einer Temperatur von 200 bis 600°C und einem Druck von 440 bis 4930 KPa umfaßt.

9. Verfahren nach Anspruch 8, worin die Regenerierung bei von 300 bis 500°C und von 790 bis 3550 KPa durchgeführt wird.

10. Verfahren nach Anspruch 8 oder 9, worin das wasserstoffenthaltende Gas im wesentlichen reiner Wasserstoff ist.

11. Verfahren nach Anspruch 8 oder 9, worin das wasserstoffenthaltende Regenerierungsgas eine Mischung von Wasserstoff und Kohlenmonoxyd mit einem Molverhältnis von Wasserstoff zu Kohlenmonoxyd von 0,2:1 bis 10:1 umfaßt.

12. Verfahren nach Anspruch 8 oder 9, worin die Regenerierung in Gegenwart von Methanol und/oder Dimethyläther bei einem Druck durchgeführt wird, der höher ist als der, der in dem Verfahren zur Umwandlung des Methanols und/oder Dimethyläthers in das Kohlenwasserstoffprodukt verwendet wird.

13. Verfahren nach einem der Ansprüche 1 bis 6, worin der Katalysator durch ein Verfahren nach einem der Ansprüche 8 bis 10 nachfolgend regeniert wird.

14. Verfahren nach einem der Ansprüche 1 bis 5 und 7, worin der Katalysator durch ein Verfahren nach einem der Ansprüche 8, 9 und 11 nachfolgend regeneriert wird.

15. Verfahren nach einem der Ansprüche 1 bis 6, worin der Katalysator durch ein Verfahren nach einem der Ansprüche 8, 9 und 12 nachfolgend regeneriert wird.

**Revendications**

1. Procédé pour transformer une charge d'alimentation comprenant du méthanol, de l'éther-oxyde de diméthyle ou un de leurs mélanges en un produit hydrocarboné riche en éthylène et en propylène, ce procédé comprenant la mise en contact de la charge d'alimentation et d'un diluant gazeux, à une température de 200 à 500°C, une pression de 440 à 3 550 kPa et une vitesse spatiale horaire des corps réactionnels organiques de 0,05 à 30, dans une zone de réaction, avec un catalyseur comprenant un aluminosilicate zéolitique cristallin ayant une structure cristalline présentant des fenêtres constituées par des pores formés par des cycles à 8 chaînons d'atomes d'oxygène, procédé caractérisé en ce que le diluant gazeux comprend un gaz contenant de l'oxygène et est introduit dans la zone de réaction à une vitesse spatiale horaire de 0,003 à 20.

2. Procédé selon la revendication 1, dans lequel la zéolite est l'érionite, l'offrétite, la chabazite, la zéolite T ou la zéolite W.

3. Procédé selon la revendication 1, dans lequel la zéolite est ZSM-34.

4. Procédé selon l'une quelconque des revendications 1 à 3, effectué à une température de 300 à 450°C et à une pression de 790 à 1 825 kPa.

5. Procédé selon l'une quelconque des revendications 1 à 4, dans lequel la charge d'alimentation comprend aussi de l'eau en une quantité d'au moins 0,25 mol/mol des corps réactionnels organiques.

6. Procédé selon l'une quelconque des revendications 1 à 5, dans lequel le diluant gazeux est de l'hydrogène essentiellement pur at dans lequel la vitesse spatiale horaire de l'hydrogène est de 0,01 à 10 et le rapport molaire de l'hydrogène aux corps réactionnels organiques se situe entre 0,5:1 et 40:1.

7. Procédé selon l'une quelconque des revendications 1 à 5, dans lequel le diluant gazeux comprend un mélange d'hydrogène et de monoxyde de carbone présentant un rapport molaire de 0,2:1 à 10:1 entre l'hydrogène et le monoxyde de carbone.

8. Procédé pour régénérer l'activité catalytique de la zéolite catalytique utilisée dans le procédé selon l'une quelconque des revendications 1 à 7, qui comprend la mise du catalyseur en contact avec un gaz contenant de l'hydrogène à une température de 200 à 600°C et à une pression de 440 à 4 930 kPa.

9. Procédé selon la revendication 8, dans lequel on effectue la régénération entre 300 et 500°C et entre 790 et 3 750 kPa.

10. Procédé selon la revendication 8 ou 9, dans lequel le gaz de régénération, contenant de l'hydrogène, est de l'hydrogène essentiellement pur.

11. Procédé selon la revendication 8 ou la revendication 9, dans lequel le gaz de régénération,

contenant de l'hydrogène, comprend un mélange d'hydrogène et de monoxyde de carbone présentant un rapport molaire, entre l'hydrogène et le monoxyde de carbone, compris entre 0,2:1 et 10:1.

12. Procédé selon la revendication 8 ou la revendication 9, dans lequel on effectue la régénération en présence de méthanol et/ou d'éther-oxyde de diméthyle à une pression supérieure à celle utilisée dans le procédé de transformation du méthanol et/ou de l'éther-oxyde de diméthyle en un produit hydrocarboné.

13. Procédé selon l'une quelconque des revendications 1 à 6, dans lequel on régénère ensuite le catalyseur par un procédé selon l'une quelconque des revendications 8 à 10.

14. Procédé selon l'une quelconque des revendications 1 à 5 et 7, dans lequel on régénère ensuite le catalyseur par un procédé selon l'une quelconque des revendications 8, 9 et 11.

15. Procédé selon l'une quelconque des revendications 1 à 6, dans lequel on regénère ensuite le catalyseur par un procédé selon l'une quelconque des revendications 8, 9 et 12.